# EUROPEAN PATENT SPECIFICATION

(11) **EP 0 428 295 B1**
(45) Date of publication and mention of the grant of the patent: **17.08.1994**
(21) Application number: 90311899.0
(22) Date of filing: 30.10.1990
(51) Int. Cl.: B01D 61/14, C12Q 1/24

(54) **Removal and identification of complex charged entities**
Entfernung und Identifizierung von komplex geladenen Einheiten
Elimination et identification d'entités complexes chargées

(30) Priority: 01.11.1989 GB 8924615
(43) Date of publication of application: 22.05.1991
(73) Proprietor: PALL CORPORATION, Glen Cove, New York 11542 (US)
(72) Inventor: Morris, Keith, Dr., Denvilles, Havant, Hants. P09 2QT (GB); Parke, Judith, Dr., Rowlands Castle, Hants. P09 6AR (GB); Morris, Debi, Denvilles, Havant, Hants. P09 2QT (GB)
(74) Representative: Knott, Stephen Gilbert

(56) References cited:
- WO-A-84/04932

## Description

The invention relates to the removal and identification of complex charged entities such as microorganisms from a liquid sample containing such entities together with entities of similar size.

In the identification of complex charged entities, the entities are often in a liquid sample that contains entities of a size similar to the entities to be identified. Examples of these are pyrogens, microorganisms such as mycoplasma, bacteria, nucleic acids, chlamydia, plasmodia, viruses, proteins and inorganic compounds such as iron oxide, and the term "complex charged entities" used herein is intended to encompass these examples. One example of a complex charged entity is the bacteria Legionella which is found in water with the water usually containing other entities.

It has been proposed to test for the presence of Legionella bacteria in the following way, using a water sample containing the bacteria. First, the sample is passed through 0.2 micrometer analysis membrane which removes the water and retains the Legionella bacteria and entities of a similar size. The membrane and the residue on the membrane are then re-suspended in water and broken-up. A portion of this sample is then added to a prefilter having a pore size which is sufficiently great to allow the Legionella bacteria to pass through but to retain as much of the unwanted entities as possible. The Legionella bacteria are then physically entrapped by a reaction membrane having a pore size smaller than the size of the bacteria and are visualised by a detection system.

This previously proposed method has a number of disadvantages. First, it requires the sample of Legionella bacteria to be concentrated using the analysis membrane. This is a difficult step to control and the variability of its outcome produces a variability in the visualisation. In addition, this concentration step significantly increases the total time required for the test.

In addition, since the prefilter also allows the passage of entities of similar size to or smaller than the Legionella bacteria, the reaction membrane is frequently blocked by such entities.

According to the invention, there is provided a method of removing and identifying complex charged entities from a liquid sample containing said complex charged entities together with unwanted entities of similar size, the method comprising passing the liquid sample through an amphoteric hydrophilic membrane having a pore size greater than the size of the complex charged entities with the pH of the liquid adjusted to provide a surface charge on the membrane such as to remove the complex charged entities from the liquid by charge capture with unwanted entities passing through the membrane as filtrate, washing the membrane with a liquid of a different pH to alter the membrane surface charge such as to release the complex charged entities and then visualizing the complex charged entities.

As used herein, the term "amphoteric" refers to the ability of the membrane surface to alter its zeta potential from a positive value to a negative value with changing pH. Thus, by using as the prefilter an amphoteric hydrophilic membrane to which the complex charged entities adhere by charge capture, unwanted entities of a similar size to the complex charged entities pass through the membrane and are removed. Accordingly, the complex charged entities are concentrated and reliable visualization is possible.

Preferably, the complex charged entities are released on to a reaction membrane having a pore size greater than the size of the complex charged entities and having a surface charge such as to cause the complex charged entities to adhere to the membrane.

In this way, a reaction membrane of increased pore size can be used, which will not become blocked.

Where the complex charged entities are of the Legionella bacteria, the amphoteric membrane preferably has a pore size of 1.2 micrometers to 5 micrometers. Most preferably, the pore size is 3 micrometers.

For Legionella bacteria, the amphoteric membrane preferably has a surface charge for removal at a pH of the liquid between 1 and 5 and a release surface charge at a pH of the liquid between 7 and 11. Most preferably, the removal surface charge is at a pH of 4 and the release surface charge is at a pH of 7.

The liquid sample may be passed through the amphoteric membrane without the application of a vacuum.

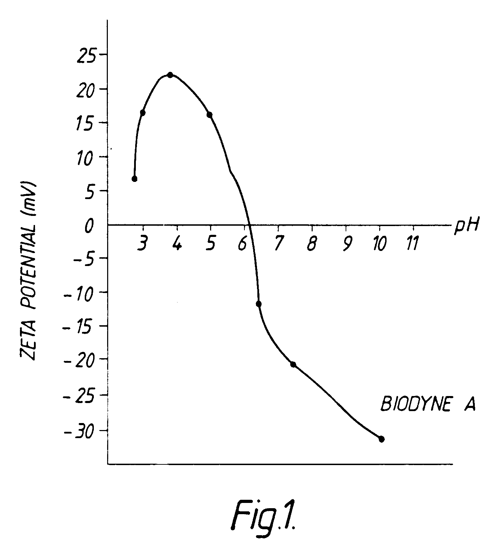

The following is a description of some examples of the invention, by way of example, and relating to the removal and identification of Legionella bacteria, reference being made to Figure 1 which is a graph of Zeta potential (surface charge) in mV against pH for specified amphoteric membranes.

The Legionella bacteria are found in cooling towers and wet air conditioning systems. Legionella bacteria are contained in the water of these towers or systems together with other unwanted entities.

It has been found that, in samples of water, the majority of such unwanted entities present are less than 2 micrometers in size. X-ray emmision spectra show that the majority of unwanted entities in those samples consist of silica. It is believed that these samples are typical as regards particle size, although the composition of the unwanted entities may vary. Cells of the Legionella bacteria have a width of between 0.4 micrometers and 0.6 micrometers and a length of between 1 micrometer and 3 micrometers.

Thus the size of non-bacterial unwanted entities in water samples containing the Legionella bacteria are similar in dimension to the dimension of the Legionella bacteria. This confirms the existence of the problem of the removal of such unwanted entities to allow concentration of the Legionella bacteria for identification.

Legionella bacteria have been identified as being negatively charged.

The following test procedure was used to test for separation of the Legionella bacteria from the unwanted entities of similar size to size of the Legionella bacteria. These tests used the following materials:-
1. A reaction filter unit fitted with a 0.45 micrometer polysulphone membrane having a pore size of 0.45 micrometers,
2. A prefilter unit fitted with an amphoteric membrane having a pore size of 3 micrometers sold under the trade name BIODYNE A by Pall Corporation. A BIODYNE A membrane is an amphoteric nylon 66 membrane which can express a range of surface charges related to the pH of the surrounding liquid. At low pH it carries a positive charge which is optimum at a pH of 4. Figure 1 is a graph of the zeta potential (in mV) against pH for BIODYNE A. In addition such membranes are hydrophilic and therefore do not require wetting before use.
3. An elution buffer of phosphate buffered saline (PBS) pH7.2 containing 150mM NaCl, 40mM Na₂HPO₄ and 8mM NaH₂PO₄.
4. Deionised water adjusted to pH4 using hydrochloric acid,
5. Deionised water adjusted to pH4 using a buffer tablet sold by British Drug Houses Limited,
6. A wash buffer containing detergent,
7. An alkaline phosphatase as an antibody enzyme conjugate,
8. Conjugate diluent buffer,
9. Nitroblue tetrazolium as a substrate solution,
10. A vacuum manifold,
11. A colour comparator for visualized Legionella bacteria,
12. Heat killed Legionella bacteria in distilled cooling tower water samples,
13. Water samples at pH4,
14. Buffer at pH4.

Where used throughout this specification in relation to membranes produced by Pall Corporation the term 'pore size' is related to absolute particle rating dependent on the thickness of the membrane and K_{L5} as described in EP-B- 0005536. An example is where an absolute particle rating of a membrane is 2 micrometers, the thickness of the membrane is 0.33 mm (0.013 inches) and the K_{L5} is 0.92 bar (13.2 p.s.i.), is equivalent to a 2 micrometer pore sized membrane. When using non-Pall membranes, pore sizes are as quoted by their manufacturer.

The test procedure was as follows:-
1. A BIODYNE A (trade mark Pall Corporation) membrane was placed in the prefilter unit and placed on the vacuum manifold. A quantity of water or buffer at a specified pH with or without Legionella cells present was added to each unit filter and left to stand for 2 to 10 minutes. A vacuum was applied for 30 seconds to pull through the sample.

The prefilter unit was then removed from the vacuum manifold and fixed to the top of a reaction filter unit. The units were then placed on the vacuum manifold.

The prefilter was then eluted by adding 5ml or 10ml of phosphate buffered saline (3) of specified pH and the vacuum was used to pull the sample through. The prefilter was then removed and discarded. After all fluid had passed through the reaction membrane, the vacuum was reapplied for 5 minutes in order to completely dry the membrane.

The reaction filter was then removed from the manifold and placed on to a cap. 1ml of enzyme conjugate was added and incubated for 30 minutes and 37°C.

The caps were then removed from the reaction units and the units were replaced on the vacuum manifold. 10ml of the wash buffer was added and incubated for 5 minutes at room temperature. The vacuum was applied until all the wash buffer was drawn through. This was repeated four times. Two final washes of 2ml per wash of distilled water were carried out, applying the vacuum immediately.

The vacuum was then left on for two minutes to dry the media. The reaction unit was then removed and again placed onto a cap. Then 1ml of substrate solution was added to each filter unit and incubated for 30 minutes at 37°C.

The substrate was poured off and the cap removed and discarded. The reaction filter unit was then washed through with 10ml of distilled water using the vacuum manifold.

The reaction membrane was then removed from the unit and visualized, with a positive result appearing as a blue precipitated spot on the middle of the membrane.

The tests were repeated with the following varying parameters:-
1. The BIODYNE A (trade mark Pall Corporation) membrane used in the prefilter unit was tested with pore sizes of 1.2 micrometers, 3 micrometers and 5 micrometers.
2. The pH of the Legionella solution added to the prefilter was varied.
3. The volume of liquid in which the Legionella bacteria were added was varied.
4. Concentration of the Legionella was varied.
5. The pH composition and volume of the elution buffer was varied.
6. The reaction membrane was varied to find an optimum reaction membrane.

The results of these tests were as follows.

Test 1. A BIODYNE A (trade mark Pall Corporation) membrane having a pore size of 3 micrometers was found to be optimum for use in the prefilter unit. It was found, however, that pore sizes between 1.2 micrometers and 5 micrometers could also be used. The surface charge of the BIODYNE A (trade mark Pall Corporation) membrane at a pH of 4 is sufficient to bind Legionella bacteria to the membrane. Other uncharged and positively charged particulate material smaller than 3 micrometers passed through the membrane. It was also found that the increased pore size of the membrane offers the potential for the sample to drip through, potentially eliminating the need for a vacuum source.

Test 2. The optimum pH of the Legionella solution applied to the prefilter was pH4, although it was found that solutions having pH's between 1 and 5 could be used.

Test 3. Up to 200ml of unconcentrated Legionella solution could be passed through the prefilter with a 100% removal of Legionella. This was due to the fact that the pore size is comparatively large, that the membrane is hydrophilic and that it is negatively charged. Accordingly, the need to concentrate the sample before pre-filtering was obviated.

Test 4. A 100% removal rate and quantitative detection of Legionella bacteria was obtained in a concentration range of from 5 x 10⁴ to 5 x 10⁸ of Legionella per sample, although Legionella were removed and detected at a concentration of 10³ Legionella per sample.
This shows that a wide range of concentrations could be dealt with.

Test 5. The optimum pH and composition of the elution buffer was phosphate buffered saline at a pH of 7 to 7.2. However, elution buffers having a pH of between 6.5 and 11 could be used.

Test 6. A reaction membrane of polysulphone having a pore size of 0.45 micrometers was blocked by unwanted entities in cooling water tower samples in at least 50% of the samples tested. This is because, although the amphoteric membrane allows the passage of unwanted entities of similar size to the Legionella bacteria, it will capture any such unwanted entities that are negatively charged. Such unwanted entities will be released with the Legionella bacteria and will tend to block the reaction membrane.

With a reaction membrane of BIODYNE B (trade mark Pall Corporation) or IMMUNODYNE I (trade mark Pall Corporation) membranes having a pore size of 3 micrometers, cooling water tower samples produced almost no blockage. This is because the pore size of such membranes is greater than the Legionella bacteria and any negatively charged unwanted entities released from the prefilter.

BIODYNE B (trade mark Pall Corporation) is a porous membrane of high purity nylon 66 which has a positive charge modified surface. IMMUNODYNE (trade mark Pall Corporation) is a porous membrane of high purity nylon 66 with a chemically preactivated surface offering a high density of covalent binding sites.

Since the membrane removes 100%, or nearly 100%, of Legionella bacteria, the visualization of the bacteria will be quantitative, so reducing the need, present in the prior art, of performing a second test for quantification.

With IMMUNODYNE I (trade mark Pall Corporation) membrane as the reaction membrane, anti-Legionella antibodies could be immobilised on the membrane as an affinity probe to capture the Legionella released from the prefilter, again avoiding blockage and allowing identification of different sub-types of the Legionella bacteria.

It will be appreciated that, although the procedures exemplified above are for the visualisation of Legionella bacteria, a similar procedure could be used for identifying other complex charged entities such as pyrogens, mycoplasma, bacteria, nucleic acids, chlamydia, plasmodia, viruses, proteins, and inorganic compounds such as iron oxide. In this case, where IMMUNODYNE I is used as the reaction membrane, the affinity probe may be other than antibodies, for example a nucleic acid sequence may be used as an affinity probe to identify viruses.

## Claims

1. A method of removing and identifying complex charged entities from a liquid sample containing said complex charged entities together with unwanted entities of a similar size, the method being characterized in that the liquid sample is passed through an amphoteric hydrophilic membrane having a pore size greater than the size of the complex charged entities with the pH of the liquid adjusted to provide a surface charge on the membrane such as to remove the complex charged entities from the liquid by charge capture with unwanted entities passing through the membrane as filtrate, the membrane then being washed with a liquid of a different pH to alter the membrane surface charge such as to release the complex charged entities and then visualizing the complex charged entities.

2. A method according to claim 1 characterized in that the complex charged entities which are released from the amphoteric membrane are added to a reaction membrane having a pore size greater than the size of the complex charged entities and having a surface charge such as to cause the complex charged entities to adhere to the reaction membrane.

3. A method according to claim 2 characterized in that the reaction membrane carries an affinity probe for identification of the complex charged entities.

4. A method according to any one of claims 1 to 3 characterized in that the complex charged entities are Legionella bacteria, the amphoteric membrane having a pore size of 1.2 micrometers to 5 micrometers.

5. A method according to claim 4 characterized in that the pore size of the amphoteric membrane is 3 micrometers.

6. A method according to claim 4 or claim 5 characterized in that the amphoteric membrane has a surface charge for removal at a pH of the liquid between 1 and 5 and a release surface charge at a pH of the liquid between 6.5 and 11.

7. A method according to claim 6 characterized in that the removal surface charge is at a pH of 4 and the release surface charge is at a pH of 7.

8. A method according to any one of claims 1 to 7 characterized in that the liquid sample is passed through the amphoteric membrane without the application of a vacuum.

## Patentansprüche

1. Verfahren zum Entfernen und Identifizieren komplex geladener Einheiten aus einer Flüssigkeitsprobe, die diese komplex geladenen Einheiten zusammen mit unerwünschten Einheiten einer ähnlichen Größe enthält, wobei das Verfahren dadurch gekennzeichnet ist, daß die Flüssigkeitsprobe durch eine amphotere hydrophile Membran mit einer Porengröße geleitet wird, die größer als die Größe der komplex geladenen Einheiten ist, wobei der pH-Wert der Flüssigkeit so eingestellt ist, daß eine Oberflächenladung auf der Membran derart geschaffen wird, daß die komplex geladenen Einheiten aus der Flüssigkeit durch Ladungsfang entfernt werden, wobei die unerwünschten Einheiten durch die Membran als Filtrat gelangen, die Membran dann mit einer Flüssigkeit eines unterschiedlichen pH-Wertes gespült wird, um die Membranoberflächenladung derart zu ändern, daß die komplex geladenen Einheiten freigegeben werden und die komplex geladenen Einheiten dann sichtbar gemacht werden.

2. Verfahren nach Anspruch 1, dadurch gekennzeichnet, daß die komplex geladenen Einheiten, die von der amphoteren Membran freigegeben werden, zu einer Reaktionsmembran hinzugefügt werden, die eine Porengröße hat, die größer als die Größe der komplex geladenen Einheiten ist und eine Oberflächenladung derart hat, daß sie bewirkt, daß die komplex geladenen Einheiten an der Reaktionsmembran haften.

3. Verfahren nach Anspruch 2, dadurch gekennzeichnet, daß die Reaktionsmembran einen Affinitätsmeßfühler zur Identifikation der komplex geladenen Einheiten trägt.

4. Verfahren nach einem der Ansprüche 1 bis 3, dadurch gekennzeichnet, daß die komplex geladenen Einheiten Legionella Bakterien sind, wobei die amphotere Membran eine Porengröße von 1,2 µm bis 5 µm hat.

5. Verfahren nach Anspruch 4, dadurch gekennzeichnet, daß die Porengröße der amphoteren Membran 3 µm ist.

6. Verfahren nach Anspruch 4 oder 5, dadurch gekennzeichnet, daß die amphotäre Membran eine Oberflächenladung zum Entfernen bei einem pH-Wert der Flüssigkeit zwischen 1 und 5 und eine Oberflächenladung zum Freigeben bei einem pH-Wert der Flüssigkeit zwischen 6,5 und 11 hat.

7. Verfahren nach Anspruch 6, dadurch gekennzeichnet, daß die Oberflächenladung zum Entfernen bei einem pH-Wert von 4 und die Oberflächenladung zum Freigeben bei einem pH-Wert von 7 ist.

8. Verfahren nach einem der Ansprüche 1 bis 7, dadurch gekennzeichnet, daß die Flüssigkeitsprobe durch die amphotere Membran ohne die Anwendung eines Vakuums geleitet wird.

## Revendications

1. Procédé d'élimination et d'identification d'entités complexes chargées provenant d'un échantillon liquide contenant lesdites entités complexes chargées avec des entités non désirées de taille similaire, le procédé étant caractérisé en ce que l'échantillon liquide est passé à travers une membrane amphotère hydrophile ayant une taille de pores supérieure à la taille des entités complexes chargées, le pH du liquide étant ajusté en sorte de fournir une charge de surface sur la membrane telle qu'elle permette d'éliminer les entités complexes chargées du liquide par capture de charge, les entités non désirées passant à travers la membrane en tant que filtrat, la membrane étant ensuite lavée avec un liquide de pH différent de façon à modifier la charge de surface de la membrane afin de libérer les entités complexes chargées, suivi d'une visualisation des entités complexes chargées.

2. Procédé selon la revendication 1, caractérisé en ce que les entités complexes chargées qui sont libérées de la membrane amphotère sont ajoutées à une membrane de réaction ayant une taille de pores supérieure à la taille des entités complexes chargées et ayant une surface de charge telle qu'elle permette de provoquer l'adhésion des entités complexes chargées à la membrane de réaction.

3. Procédé selon la revendication 2, caractérisé en ce que la membrane de réaction porte une sonde d'affinité pour l'identification des entités complexes chargées.

4. Procédé selon l'une quelconque des revendications 1 à 3, caractérisé en ce que les entités complexes chargées sont des bactéries Legionella, la membrane ayant une taille de pores de 1,2 micromètres à 5 micromètres.

5. Procédé selon la revendication 4, caractérisé en ce que la taille de pores de la membrane amphotère est de 3 micromètres.

6. Procédé selon la revendication 4 ou la revendication 5, caractérisé en ce que la membrane amphotère a une charge de surface pour l'élimination à un pH du liquide compris entre 1 et 5 et une charge de surface pour la libération à un pH du liquide compris entre 6,5 et 11.

7. Procédé selon la revendication 6, caractérisé en ce que la charge de surface pour l'élimination est à un pH de 4 et la charge de surface pour la libération est à un pH de 7.

8. Procédé selon l'une quelconque des revendications 1 à 7, caractérisé en ce que l'échantillon liquide est passé à travers une membrane amphotère sans application de vide.
